# EUROPÄISCHE PATENTSCHRIFT

(11) **EP 0 686 030 B1**
(45) Veröffentlichungstag und Bekanntmachung des Hinweises auf die Patenterteilung: **28.02.2001**
(21) Anmeldenummer: 95903221.0
(22) Anmeldetag: 23.12.1994
(51) Int. Cl.: A61K 9/16, A61K 39/39

(54) **Mit Antigen beladene Mikropartikel und pharmazeutische Zubereitungen, die diese Mikropartikel enthalten.**
Microparticles loaded with antigen and pharmaceutical compositions containing these microparticles.
Microparticules chargées d'antigène et compositions pharmaceutiques contenant ces microparticules.

(30) Priorität: 23.12.1993 CH 384993
(43) Veröffentlichungstag der Anmeldung: 13.12.1995
(73) Patentinhaber: Gander, Bruno, 6405 Immensee (CH); Corradin, Giampietro, 1000 Lausanne (CH); Men, Ying, 8037 Zürich (CH); Thomasin, Claudio, 8239 Dörflingen (CH); Merkle, Hans Peter, 8049 Zürich (CH)
(72) Erfinder: Gander, Bruno, 6405 Immensee (CH); Corradin, Giampietro, 1000 Lausanne (CH); Men, Ying, 8037 Zürich (CH); Thomasin, Claudio, 8239 Dörflingen (CH); Merkle, Hans Peter, 8049 Zürich (CH)
(74) Vertreter: Goth, Helmut, Dr.
(86) Internationale Anmeldenummer: CH9400242
(87) Internationale Veröffentlichungsnummer: WO9517167

(56) Entgegenhaltungen:
- WO-A-91/07171
- GB-A- 2 189 143
- POLYMERS FOR ADVANCED TECHNOLOGIES, Bd. 3,Nr. 6, Oktober 1992 CHICHESTER (GB), Seiten 351-357, XP 000324943 S. AMSELEM ET AL. 'polymeric biodegradable lipospheres (tm) as vaccine delivery systems'
- MANUFACTURING CHEMIST, Bd. 63,Nr. 1, Januar 1992 WOOLWICH (GB), Seiten 23-26, XP 000301440 K.J. STEFFENS ET AL. 'o/w emulsions as carriers for micronised drug particles'
- VACCINE, Bd. 10,Nr. 10, August 1992 LONDON (GB), Seiten 714-720, I. ESPARZA ET AL. 'parameters affecting the immunogenicity of microencapsulated tetanus toxoid'

## Beschreibung

Die vorliegende Erfindung betrifft mit Antigen beladene Mikropartikel aus biokompatiblem und bioabbaubarem Material, sowie pharmazeutische Zubereitungen zur parenteralen Applikation in Form einer Suspension dieser mit Antigen beladenen Mikropartikel in einem biokompatiblen und bioabbaubaren Dispersionsmedium. Die vorliegende Erfindung betrifft insbesondere mit synthetischen, schwach immunogenen Antigenen beladene Mikropartikel aus biokompatiblem und bioabbaubarem Material, sowie parenteral applizierbare pharmazeutische Zubereitungen, welche diese mit Antigen beladenen Mikropartikel enthalten.
Unter synthetischen, schwach immunogenen Antigenen werden nachfolgend Verbindungen mit Peptid- bzw. Proteinstruktur verstanden, welche entweder chemisch oder mittels rekombinanter DNA-Technologie hergestellt werden und die nach parenteraler Verabreichung in wässeriger Lösung oder als Aluminiumadsorbat eine nur unbedeutende immunologische Antwort mit sehr niedrigen Antikörpertitern und fehlender oder nur geringer T-Zell Proliferation auslösen. Nachfolgend soll diese Gruppe von Antigenen der Einfachheit halber als synthetische Antigene bezeichnet werden. Definitionsgemäss ist also die Immunantwort auf die hier beschriebenen synthetischen Antigene vernachlässigbar, wenn diese in wässeriger Lösung verabreicht werden. Als experimentelle Referenzzubereitung wird Inkomplettes Freund's Adjuvans (IFA) verwendet. IFA ist eine Wasser in Öl (W/O) Zubereitung, welche bekannterweise sowohl die humorale wie zelluläre Immunantwort stimuliert. Wegen starken unerwünschten Nebeneffekten darf IFA jedoch nur zu Versuchszwecken verwendet werden. Unter dem Begriff Impfstoff werden nachfolgend Formulierungen verstanden, welche zusätzlich zum Antigen Stoffe enthalten, die ihrerseits reine Hilfsstoffunktion oder eine immunpotenzierende Funktion oder gar eine Kombination beider Funktionen ausüben. Reine Hilfsstoffe sind beispielsweise Wasser zur Auflösung des Antigens für die parenterale Verabreichung, antimikrobielle, isotonisierende und pH-stabilisierende Hilfsstoffe. Immunpotenzierende Stoffe werden oft auch als Adjuvantien bezeichnet, worunter beispielsweise unlösliche Aluminiumsalze (-phosphate, -hydroxide), gewisse Lipopolysaccharide, Muramylpeptide, Trehalose-Verbindungen, verschiedene Cytokine wie Interleukin 1, lipophile Blockcopolymere (Poloxamere) fallen. Adjuvanseigenschaften besitzen jedoch auch die experimentelle Referenzzubereitung Inkomplettes Freund's Adjuvans und verschiedene noch in der Entwicklung sich befindliche Impfstoff-Darreichungsformen wie Liposomen, Emulsionen, Nanokapseln. Diese Darreichungsformen bewirken nicht nur die Bildung eines Antigendepots in vivo, sondern besitzen auch immunstimulierende Eigenschaften.

Die Entwicklung neuer Impfstoffe und die Verbesserung bestehender Impfstoff-Formulierungen hat in den letzten Jahren an Bedeutung und Dringlichkeit gewonnen (E. Eppstein et al., New adjuvants for vaccines containing purified protein antigens, Advances in Drug Delivery Review 4, 233-253, (1990)). Die Herstellung synthetischer Antigene wie auch die Entwicklung geeigneter Adjuvansformulierungen und Darreichungsformen, welche die Immunogenität von schwach immunogenen Verbindungen erhöhen, stehen dabei im Vordergrunde. Die Entwicklung neuer Antigene hat einerseits Krankheiten zum Ziel, gegen die es noch keine oder nur ungenügend wirksame Impfstoffe gibt wie beispielsweise AIDS, Malaria, Tuberkulose, Cholera, Hepatitis A, Krebserkrankungen; andererseits gehen die Bemühungen dahin, die in den traditionellen Impfstoffen enthaltenen Antigene wie inaktivierte Viren, Bakterien oder Toxoide, durch einfacher zu produzierende und zu reinigende und besser charakterisierbare niedermolekulare Peptide und Proteine zu ersetzen, welche die antigenen Bereiche der eigentlichen Infektionserreger in ihrer Struktur aufweisen. Solche antigenen Peptide und Proteine können biochemisch oder durch rekombinante DNA-Technologie in hoher Reinheit gewonnen werden. Diese neue Generation von synthetischen Antigenen besitzen in ihrer chemischen Struktur Peptidsequenzen (Epitope), welche antigen-spezifische T_{H}- (Helfer), T_{C}- (cytotoxisch) und B-Lymphozyten stimulieren. Dabei können die sogenannten T_{H}-, T_{C}-und B-Zell-Epitope je einzeln vorliegen oder kovalent zu einem chimeren B-T-Epitop verknüpft werden. Da diese gentechnologisch oder chemisch hergestellten Antigene im allgemeinen niedrige Molekulargewichte von zirka 500 - 2'000 aufweisen, ist ihre Immunogenität im Gegensatz zu Toxoiden mit Molekulargewichten von 50'000 bis 150'000 oder im Gegensatz zu partikulären Antigenen wie inaktivierten Viren und anderen Mikroorganismen sehr schwach.

Bisher bekannte Strategien zur Immunogenitätspotenzierung von synthetischen Antigenen beruhen darauf, in einem ersten Schritt das Molekulargewicht dieser Antigene durch kovalente Verknüpfungen zu erhöhen, und in einem zweiten Schritt diese höher molekularen Konstrukte in immunpotenzierende Formulierungen einzubringen.

Es ist bekannt, dass die Erhöhung des Molekulargewichtes dadurch erreicht werden kann, dass das synthetische Antigen kovalent an hochmolekulare Trägerproteine wie beispielsweise Diphtherie- und Tetanus-Toxoid, Rinderserumalbumin, Napfschnekken-Haemocyanin gebunden wird. Nachteilig an den Antigen-Trägerprotein-Konstrukten sind der Einsatz von sehr teuren und relativ unreinen Trägerproteinen aus Fremdorganismen, die Notwendigkeit von reaktiven und relativ toxischen Agenzien zur kovalenten Verknüpfung von Antigen und Trägerprotein, und die Schwierigkeit der Reinigung, sowie die Identitäts- und Reinheitsprüfung dieser Verbindungen. Es wurde andererseits auch vorgeschlagen, das Molekulargewicht von B-T-Epitopen dadurch zu erhöhen, dass diese selbst in einer Art Aststruktur kovalent zu Multimeren verknüpft werden (J.P. Tam, Y.-A. Lu, Proceedings of the National Academy of Sciences of the USA 86, 9084-9088 (1989)) .Diese Konstrukte werden als Multiple Antigen Peptide, kurz MAP, bezeichnet.

Weiterhin ist bekannt, dass die Kombination eines B- und T_{H}-Epitops essentiell ist für das Zustandekommen einer Antikörperbildung und dass die Kombination eines T_{C}-Epitops mit einem T_{H}- Epitop die cytotoxische Lymphozyten-Antwort, auch CTL-Antwort genannt, nach Verabreichung in IFA verbessern kann (C. Widmann et al., J. Immunol. Methods 155, 95-99 (1992)).

In EP-A-0 513 861 werden verschiedene immunpotenzierende Formulierungen für solche schwach immunogenen Antigene und deren höhermolekularen Konstrukte beschrieben. Dazu gehören in erster Linie Immunstimulantien enthaltende O/W-Emulsionen. Nachteilig an diesen grobdispersen bzw. kolloiddispersen Systemen sind ihre inherente thermodynamische Instabilität, die sich bei Lagerung in Koaleszenzerscheinungen widerspiegeln kann. Weiterhin unterliegen die Komponenten solcher flüssig-dispersen Formulierungen chemischen Veränderungen wie Oxydation und Hydrolyse. Zudem benötigen die beschriebenen Formulierungen meist Immunstimulantien wie Muramylpeptide, die toxikologisch nicht ganz unbedenklich sind. Schliesslich zeigen diese Formulierungen keinerlei Langzeiteffekt. Um einen Impfschutz über mehrere Jahre zu erzielen ist es deshalb notwendig diese Impfstoff-Formulierungen nach einem festgelegten Impfplan drei-bis viermal zu injizieren (sogenannte "Booster"-Injektionen).

Im weiteren ist aus der internationalen Patentanmeldung WO-92/19263 ein System zur Immunpotenzierung von Antigenen bekannt, welches sogenannte biodegradierbare Mikrosphären, auch Mikrokapseln oder Mikropartikel genannt, verwendet. Nachteilig an diesem Verfahren ist, dass die Immunpotenzierung hauptsächlich in der gastrointestinalen Mukosa beobachtet wird und deshalb nur für relativ wenige Krankheitserreger (sog. enteropathogene Mikroorganismen) Wirkung zeigen dürfte. Die Tatsache, dass die Mikropartikel zudem in den Zwölffingerdarm verabreicht werden und nicht peroral eingegeben oder eingenommen werden können, schliesst eine praktische Anwendung, zumindest beim Menschen, aus. Ausserdem scheint nach EP-A-0 333 523 ein ausgewogenes Mass an feinen (1 - 10 µm) und grobkörnigeren (20 - 50 µm) Mikropartikel eine wichtige Voraussetzung für den immunpotenzierenden Effekt zu sein. Diese Anforderungen an den Korngrössenbereich der Mikropartikel stellen einen Mehraufwand bei der Herstellung und Aufarbeitung der Mikropartikel dar, was nachteilig erscheint.
Ein wesentlicher Nachteil der in WO 92/19263 und in EP-A-0 333 523 beschriebenen pharmazeutischen Zubereitungen besteht darin, dass diese keine cytotoxische T-Zell-Antwort bewirken und somit gegen virale und parasitäre Infektionskrankheiten oder Tumorerkrankungen keinen Schutz bieten können.

Weiterhin sind in der internationalen Patentanmeldung WO 91/07171 und in Polymers for Advanced Technologies, Bd. 3, Nr. 6, Oktober 1992, Seiten 351-357, pharmazeutische Zubereitungen beschrieben, die aus sogenannten Liposphären bestehen, in die ein Antigen eingebettet wurde. Gemäss diesen Publikationen bewirken diese Zubereitungen nach parenteraler Verabreichung eine Antikörper- und T_{H}-Zell-Antwort. Auch diese in WO 91/07171 und in Polymers for Advanced Technologies, Bd. 3, Nr.6, Oktober 1992, Seiten 351-357, beschriebenen pharmazeutischen Zubereitungen bewirken keine cytotoxische T-Zell-Antwort und können somit gegen virale und parasitäre Infektionskrankheiten oder Tumorerkrankungen keinen Schutz bieten.

Aufgabe der Erfindung ist es, synthetische, schwach immunogen wirkende Antigene enthaltende, parenteral applizierbare pharmazeutische Zubereitungen bereitzustellen, die eine starke und lange anhaltende cytotoxische T-Zell-Antwort bewirken und die somit gegen virale und parasitäre Infektionskrankheiten und gegen Tumorerkrankungen Schutz bieten können.

Es wurde überraschenderweise gefunden, dass man die Immunantwort von an sich schwach immunogenen (synthetischen) cytotoxischen T-Zell T_{C}-Epitopen wesentlich verstärken kann, wenn man diese T-Zell (T_{C})-Epitope in Mikropartikel aus biokompatiblen und bioabbaubaren Biopolymeren einbettet, die in wässerigen Medien und physiologischen Flüssigkeiten benetzbar, quellbar und unlöslich sind, die so erhaltenen mit Antigen beladenen Mikropartikel in eine injizierbare Formulierung überführt und parenteral appliziert.

Gegenstand der vorliegenden Erfindung sind daher mit Antigen beladene Mikropartikel aus biokompatiblem und bioabbaubarem Material, die dadurch gekennzeichnet sind, dass sie mindestens ein cytotoxisches T_{C}-Epitop enthalten und das biokompatible und bioabbaubare Material ein in wässerigen Medien und physiologischen Flüssigkeiten quellbares benetzbares unlösliches Biopolymeres ist.

Ein weiterer Gegenstand der vorliegenden Erfindung ist eine pharmazeutische Zubereitung zur parenteralen Applikation in Form einer Suspension von mit Antigen beladenen Mikropartikeln aus biokompatiblem und bioabbaubarem Material in einem biokompatiblen und bioabbaubaren Dispersionsmedium, die dadurch gekennzeichnet ist, dass
a) die Mikropartikel mindestens ein cytotoxisches T-Zell-Epitop enthalten,
b) die Mikropartikel aus mindestens einem in wässerigen Medien und physiologischen Flüssigkeiten quellbaren benetzbaren unlöslichen Biopolymeren bestehen, und
c) das biokompatible und bioabbaubare Dispersionsmedium eine wässerige oder ölige Lösung von Lecithin, eine wässerig-ölige Emulsion von Lecithin im Konzentrationsbereich von 0,1 bis 20%, oder eine aus einer Wasser-, Öl-, Tensid- und Kotensid-Komponente bestehende Mikroemulsion ist.

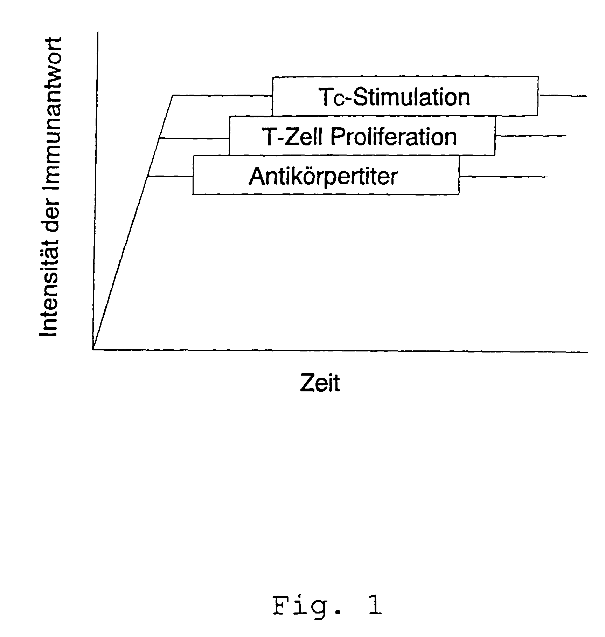

Weitere spezielle Merkmale der vorliegenden Erfindung sind aus der nachfolgenden Beschreibung des Verfahrens zur Herstellung der erfindungsgemässen mit Antigen beladenen Mikropartikel und der diese Mikropartikel enthaltenden pharmazeutischen Zubereitung und deren Anwendung, sowie aus Figuren 1 bis 7 ersichtlich. Es zeigen:
- Fig. 1: Schematische Darstellung der mit den erfindungsgemässen Zubereitungen erreichbaren Immunpotenzierung
- Fig. 2: Antikörpertiter nach einmaliger Verabreichung von MAP enthaltenden, schnell freigebenden Mikrokapseln und einer IFA-Formulierung
- Fig. 3: Antikörpertiter nach einmaliger Verabreichung von MAP enthaltenden, langsam freigebenden Mikrokapseln und einer IFA-Formulierung
- Fig. 4: Antikörpertiter nach einmaliger Verabreichung einer MAP enthaltenden Mischung von schnell und langsam freigebenden Mikrokapseln und einer IFA-Formulierung
- Fig. 5: Antikörpertiter nach dreimaliger Verabreichung von MAP enthaltenden, schnell freigebenden Mikrokapseln und einer IFA-Formulierung
- Fig. 6: Proliferative T-Lymphozyten-Antwort nach einmaliger Verabreichung verschiedener MAP enthaltender Mikrokapseln und einer IFA-Formulierung
- Fig. 7: T_{C}-Antwort nach einmaliger Verabreichung einer Mischung schnell freigebender Mikrokapseln, die jeweils ein synthetisches T_{C}-Antigen und ein entsprechendes MAP enthalten

### 1. Einbetten von synthetischem Antigen in bioabbaubare Mikropartikel

Ausgangspunkt des Verfahrens zur Herstellung der erfindungsgemässen Zubereitungen sind synthetische Antigene gemäss Definition in der Einleitung dieser Patentschrift, die in ihrer bekannten chemischen Struktur mindestens ein definiertes und vom Immunsystem erkennbares Epitop eines pathogenen Mikroorganismus enthalten. Dabei kann es sich beim Epitop sowohl um ein B-Zell-Epitop, ein T_{H}-Zell-Epitop, ein T_{C}-Epitop oder eine beliebige Mischung dieser Epitope handeln. Bevorzugterweise bilden die sogenannten Multiplen Antigen Peptide (MAP), allein oder in Kombination mit einem T_{C}-Epitop, den Ausgangspunkt des Verfahrens. Die Herkunft der Epitope schliesst Bakterien, Viren, Protozoen und Tumorzellen ein. Erfindungsgemäss wird das synthetische Antigen in bioabbaubare Mikropartikel eingebettet. Erfindungswesentlich ist, dass zur Herstellung der bioabbaubaren Mikropartikel Biopolymere mit spezifischen physikalisch-chemischen Eigenschaften ausgewählt werden. Massgebliche Eigenschaften sind die Benetzbarkeit, die Unlöslichkeit, die Quellbarkeit und die Bioabbaubarkeit der Biopolymere und der daraus hergestellten sphärischen Mikropartikel in wässerigen Medien und physiologischen Flüssigkeiten. Das Ausmass der Quellbarkeit der Biopolymere sowie deren Bioabbauzeit bestimmen massgeblich die Freisetzungskinetik der Antigene aus den Mikrokapseln. Überraschenderweise wurde nun gefunden, dass diese Freigabekinetik auch den zeitlichen Verlauf der Immunantwort beeinflusst. Beispiele solcher Biopolymere mit unterschiedlicher Benetzbarkeit, Quellbarkeit und Bioabbauzeit sind Poly(milchsäure), Poly(milch-co-glykolsäure), Poly(hydroxybuttersäure), Poly(hydroxybutter-co-valeriansäure), Poly(caprolacton). Die Einbettung des synthetischen Antigens in das Biopolymer erfolgt mittels verschiedener bekannter Verfahren wie Sprühtrocknung, Lösungsmittel-Verdampfung oder Koazervation. Dabei resultieren antigenbeladene, sphärische Mikropartikel in der Grössenordnung von 1 bis 200 µm.

### 2. Suspendieren im Dispersionsmedium

Die erfindungsgemässen antigenbeladenen Mikropartikel werden in einem zweiten Schritt in ein Dispersionsmedium eingebracht, das für die parenterale Verabreichung der Mikropartikel geeignet ist. Erfindungswesentlich ist dabei, dass das Dispersionsmedium biokompatibel und bioabbaubar ist, und zusätzlich für die Potenzierung der Immunantwort vorteilhafte Eigenschaften besitzt. Solche vorteilhaften Dispersionsmedien sind beispielsweise wässrige und ölige Lösungen von Lecithin oder wässrig-ölige Emulsionen mit Lecithin in einem Konzentrationsbereich von 0,1 - 20 %, bevorzugterweise von 2 - 10 %. Weitere geeignete Dispersionsmedien sind sogenannte Mikroemulsionen, die aus einer Wasser-, Öl-, Tensid- und Kotensid-Komponente bestehen. Hierzu werden biokompatible und bioabbaubare Substanzen wie beispielsweise natürliche und synthetische Mono-, Di- und Triglyceride, Lecithin, Poloxamere und Polysorbate verwendet. Die genannten Dispersionsmedien zeichnen sich durch überraschend gute Benetzungs- und Suspendiereigenschaften für die bioabbaubaren Mikropartikel aus. Diese Benetzungs- und Suspendiereigenschaften sind beispielsweise wesentlich besser als jene der üblicherweise verwendeten Dispersionsmedien, wie Carboxymethylcellulose oder Polysorbat. Die Dispergierung der Mikropartikel im Dispersionsmedium kann durch einfaches Schütteln erfolgen, wobei eine injizierbare Zubereitung entsteht.

### 3. Verabreichen

Die antigenbeladenen und im Dispersionsmedium suspendierten Mikropartikel werden parenteral verabreicht, wobei diese Verabreichung einmalig oder mehrmalig in bestimmten Zeitabständen erfolgen kann. Letztere Verabreichungsart ist unter dem Begriff 'Boosten' bekannt. Die 1. und 2. Booster-Dosis kann beispielsweise 1 - 4 Wochen und 3 - 6 Monate nach der ersten Injektion verabreicht werden. Nach einmaliger oder mehrmaliger Verabreichung der erfindungsgemässen Formulierungen wird eine potenzierte, mehrere Monate anhaltende Immunantwort ausgelöst.

### 4. Bewirken der potenzierten Immunantwort

Die Potenzierung der Immunantwort wird im allgemeinen nach einmaliger, ausnahmsweise jedoch auch nach dreimaliger parenteraler Verabreichung der erfindungsgemässen mit synthetischen Antigenen beladenen Mikropartikel in BALB/c Mäusen gemessen. Als synthetische Modell-Antigene wird ein MAP, aufgebaut aus einem universellen T_{H}-Epitop des Tetanus Toxins (Sequenz 947-967) und einem B-Zell Epitop der repetitiven Region des Circumsporozoit Proteins von Plasmodium berghei, und ein T_{C}-Epitop von Plasmodium berghei Circumsporozoit-Protein (Sequenz 252-260) verwendet (S. Demotz et al., J. of Immunology, 142, 394-402 (1989); P. Romero et al., Nature 341, 323 (1989); J.L. Weber et al., Exp. Parasitology 63, 295 (1987)). Die Intensität und die Dauer der Immunpotenzierung wird anhand der spezifischen Antikörpertiter, der T-Lymphozyten-Proliferation und der spezifischen, cytotoxischen T-Lymphozyten-Aktivität gemessen. Diese drei Parameter werden nach bekannten immunologischen Methoden bestimmt.

Fig. 1 illustriert schematisch relevante Parameter der mit den erfindungsgemässen Zubereitungen erzielten Immunpotenzierung. Eine Immunpotenzierung bedeutet in der vorliegenden Patentschrift, dass die Intensität der immunologischen Antwort im zeitlichen Verlauf auf ein verabreichtes synthetisches Antigen auf den Ebenen des Antikörpertiters, der T-Zell-Proliferation und der T_{C}-Stimulation gegenüber einer wässrigen Antigenlösung potenziert und gegenüber einer IFA-Formulierung in vergleichbarem oder erhöhtem Masse potenziert ist.

Daraus ergibt sich die Möglichkeit durch Mischung von Biopolymeren mit unterschiedlicher Benetzbarkeit, Quellbarkeit und Bioabbauzeit sowohl die humorale Antikörper-Antwort wie auch die zelluläre T-Lymphozyten Antwort in einem Masse zu potenzieren, das vergleichbar oder sogar höher ist als die mit Inkomplettem Freund's Adjuvans erzielte Potenzierung. Zudem sind die Immunantworten gemäss diesem Verfahren zeitlich steuerbar und gegenüber IFA und wässeriger Lösung um mehrere Wochen verlängert.

Mit den erfindungsgemässen Zubereitungen wird aufgrund der massgeschneiderten Eigenschaften der verwendeten, mit Antigen beladenen Mikropartikel eine gezielte und in ihrem zeitlichen Verlaufe steuerbare Potenzierung der humoralen und zellulären Immunantwort auf synthetische Antigene, insbesondere auf die sogenannten MAP möglich. Die erfindungsgemässen, mit Antigen beladenen Mikropartikel besitzen überdies den ausserordentlichen Vorteil, dass nebst der gezielten und potenzierten Stimulation von B- und T_{H}-Lymphozyten auch cytotoxische T-Lymphozyten stimuliert werden können, wodurch insbesondere auch eine Immunisierung gegen Viren, Protozoen und Tumorzellen erfolgreich durchgeführt werden kann. Diese cytotoxische T-Lymphozyten Stimulation konnte hier überraschenderweise zum ersten Mal gezeigt werden. Im Gegensatz zu der in EP-A-0 333 523 und WO-92/19263 beschriebenen Immunpotenzierung ist diese hier in erster Linie systemisch, d.h. nicht mukosal, und kann sowohl in der Intensität wie der Dauer, bzw. im zeitlichen Verlauf, gesteuert werden. Zudem ist für die Immunpotenzierung keine enge, genau definierte Partikelgrössenverteilung notwendig, was technologische Vorteile mit sich bringt.

Die erfindungsgemässen, mit Antigen beladene Mikropartikel enthaltenden Zubereitungen finden Anwendung bei der Immunisierung von Mensch und Tier gegen Krankheiten, die durch Bakterien, Viren, Protozoen und Tumorzellen verursacht werden. Besonders die Immunisierung gegen Viren, Protozoen und Tumorzellen, die mit herkömmlichen Impfstoffen nur unbefriedigend, d.h. ungenügend und unter Inkaufnahme von unerwünschten Nebenwirkungen, erreicht werden kann, stellt eine Hauptanwendung der erfindungsgemässen, mit Antigen beladenen Mikropartikel und der diese Mikropartikel enthaltenden pharmazeutischen Zubereitungen dar. Die Stimulierung der cytotoxischen T-Zellen durch die erfindungsgemässen Zubereitungen sowie die über eine längere Zeitperiode anhaltende Immunantwort bilden die Basis für diese Anwendung.

Beispiel 1 beschreibt die Potenzierung der Antikörperantwort auf das verzweigte Multiple Antigen Peptid mit der Bezeichnung P30B2, welches aus einem universellen T_{H}-Zell Epitop des Tetanus Toxins (Sequenz 947-967) und einem B-Zell Epitop der repetitiven Region des Circumsporozoiten Proteins von Plasmodium berghei aufgebaut ist: 0,02 g P30B2 wurden in 2,00 g Wasser gelöst und diese Lösung wurde anschliessend mit Hilfe eines Ultraschallgenerators in einer Lösung von 2,0 g Poly(d,l-milchsäure-co-glykolsäure) 50:50 (Resomer 502, Boehringer Ingelheim) in 40,0 g Dichlormethan dispergiert. Mittels Sprühtrocknung wurden aus dieser Dispersion sphärische Mikropartikel (RG502) hergestellt. Mit Antigen beladene Mikropartikel wurden anschliessend in einer 5% sterilen Lösung von Eilecithin (Ovothin 170, Lukas Meyer, D-Hamburg) durch Schütteln suspendiert. Diese Suspension wurde einer Gruppe von 8 BALB/c Mäusen zu je 0,5 ml subcutan injiziert. Die pro Maus injizierte Menge Antigen betrug 30 µg. Als Vergleich wurde eine zweite Gruppe von 8 BALB/c Mäusen mit der gleichen Menge Antigen in Inkomplettem Freund's Adjuvans (IFA) immunisiert. Die Antikörpertiter wurden mittels ELISA bestimmt.

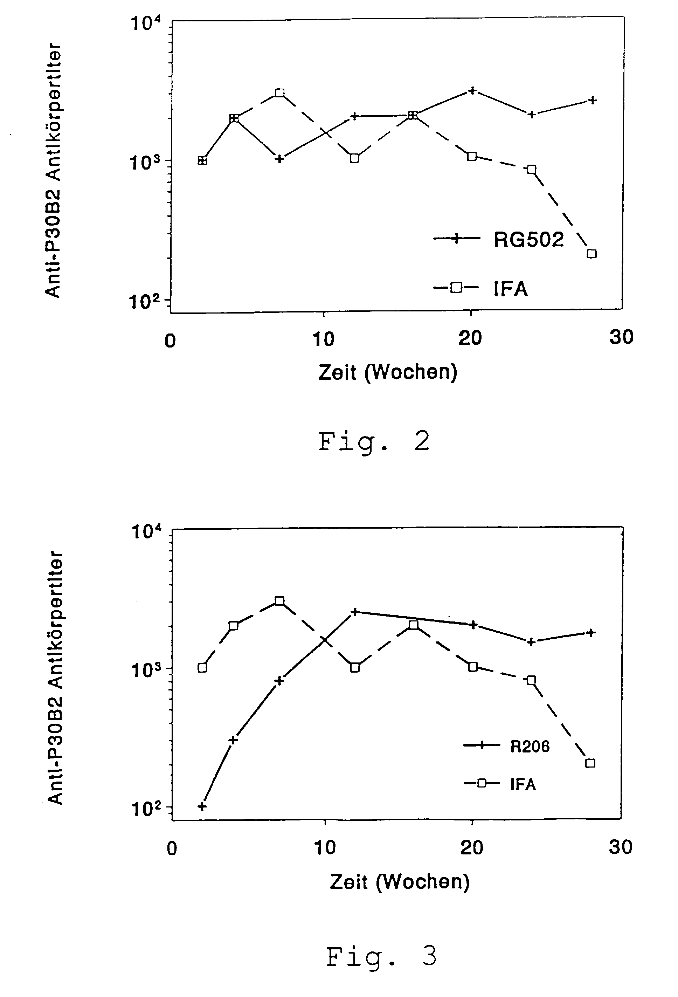

Fig. 2 zeigt den zum Beispiel 1 gehörenden zeitlichen Verlauf der Immunpotenzierung durch RG502 im Vergleich zu IFA. Die mit RG502 und IFA erzielten Antikörpertiter sind während der ersten 15 Wochen nach Immunisierung untereinander vergleichbar. Danach fallen die durch IFA induzierten Titer ab, während die mit den Mikrokapseln induzierten Titer während mindestens 28 Wochen konstant bleiben. Mit einem hydrophilen, stark quellbaren, schnell freigebenden und schnell bioabbbaubaren Biopolymeren wie dem PLGA 50:50 werden Antikörpertiter von 1 - 2 · 10³ bereits zwei Wochen nach Verabreichung erreicht und bleiben über eine Zeitdauer von mindestens 28 Wochen konstant. Im Gegensatz dazu fallen die nach einmaliger Verabreichung einer IFA-Zubereitung gemessenen Titer bereits nach 15 Wochen ab und liegen nach 28 Wochen nur noch bei 2·10².

Beispiel 2 beschreibt die Potenzierung der Antikörperantwort auf das verzweigte Multiple Antigen Peptid mit der Bezeichnung P30B2 (gemäss Beispiel 1), welches aus einem universellen T_{H}-Zell Epitop des Tetanus Toxins (Sequenz 947-967) und einem B-Zell Epitop der repetitiven Region des Circumsporozoiten Proteins von Plasmodium berghei aufgebaut ist: 0,02 g P30B2 wurden in 2,00 g Wasser gelöst und diese Lösung wurde anschliessend mit Hilfe eines Ultraschallgenerators in einer Lösung von 2,0 g Poly(d,l-milchsäure) (Resomer 206, Boehringer Ingelheim) in 40,0 g Dichlormethan dispergiert. Mittels Koazervation, induziert durch Silikonölzugabe, wurden aus dieser Dispersion sphärische Mikropartikel (R206) hergestellt. Mit Antigen beladene Mikropartikel wurden anschliessend in einer 5% sterilen Lösung von Eilecithin (Ovothin 170, Lukas Meyer, D-Hamburg) durch Schütteln suspendiert. Diese Suspension wurde einer Gruppe von 8 BALB/c Mäusen zu je 0,5 ml subcutan injiziert. Die pro Maus injizierte Menge Antigen betrug 30 µg. Als Vergleich wurde eine zweite Gruppe von 8 BALB/c Mäusen mit der gleichen Menge Antigen in Inkomplettem Freund's Adjuvans (IFA) immunisiert. Die Antikörpertiter wurden mittels ELISA bestimmt.

Fig. 3 zeigt den zum Beispiel 2 gehörenden zeitlichen Verlauf der Immunpotenzierung durch R206 im Vergleich zu IFA. Die mit dem hydrophoben, schwach quellbaren, langsam freigebenden und langsam bioabbaubaren R206 erzielten Antikörpertiter steigen während der ersten 12 Wochen kontinuierlich an und erreichen dann das Niveau, das mit IFA bereits 2 Wochen nach Immunisierung erzielt wurde. Während die IFA-Titer nach ungefähr 15 Wochen wieder stetig abfallen, bleiben die R206-Titer über einen Zeitraum von mindestens 28 Wochen konstant.

Beispiel 3 beschreibt die Potenzierung der Antikörperantwort auf das verzweigte Multiple Antigen Peptid mit der Bezeichnung P30B2 (gemäss Beispiel 1), welches aus einem universellen T_{H}-Zell Epitop des Tetanus Toxins (Sequenz 947-967) und einem B-Zell Epitop der repetitiven Region des Circumsporozoiten Proteins von Plasmodium berghei aufgebaut ist: P30B2 wurde analog Beispiel 1 in Poly(d,l-milchsäure-co-glykolsäure) 75:25 (Resomer RG752, Boehringer Ingelheim) eingebaut und zu sphärischen Mikropartikeln (RG752) verarbeitet. Identische Mengen P30B2 enthaltende Mikropartikel RG752, RG502 (aus Beispiel 1) und R206 (aus Beispiel 2) wurden in einer 5% sterilen Lösung von Eilecithin (Ovothin 170, Lukas Meyer, D-Hamburg) durch Schütteln suspendiert. Diese Suspension wurde einer Gruppe von 8 BALB/c Mäusen zu je 0,5 ml subcutan injiziert. Die pro Maus injizierte Menge Antigen betrug 30 µg. Als Vergleich wurde eine zweite Gruppe von 8 BALB/c Mäusen mit der gleichen Menge Antigen in Inkomplettem Freund's Adjuvans (IFA) immunisiert. Die Antikörpertiter wurden mittels ELISA bestimmt.

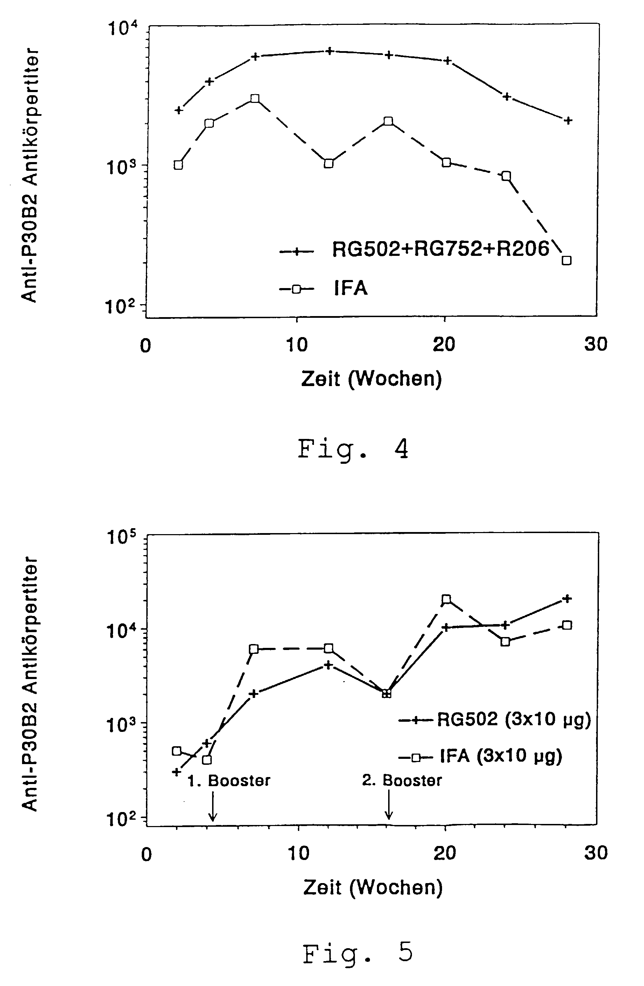

Fig. 4 zeigt den zum Beispiel 3 gehörenden zeitlichen Verlauf der Immunpotenzierung durch eine Mischung von RG502, RG752 und R206 im Vergleich zu IFA. Die mit dieser aus schnell und langsam freigebenden Biopolymeren bestehenden Mikrokapselmischung erzielten Antikörpertiter steigen schnell an und erzielen bereits 2 Wochen nach Immunisierung ein Niveau, das um einen Faktor 2,5 höher liegt als die mit IFA induzierten Antikörpertiter. Während die IFA-Titer nach ungefähr 15 Wochen wieder stetig abfallen, bleiben die mit der Mikrokapselmischung erzielten Titer über einen Zeitraum von mindestens 28 Wochen relativ konstant.

Beispiel 4 beschreibt die Potenzierung der Antikörperantwort auf das verzweigte Multiple Antigen Peptid mit der Bezeichnung P30B2 (gemäss Beispiel 1), welches aus einem universellen T_{H}-Zell Epitop des Tetanus Toxins (Sequenz 947-967) und einem B-Zell Epitop der repetitiven Region des Circumsporozoiten Proteins von Plasmodium berghei aufgebaut ist: P30B2 wurde analog Beispiel 1 in Poly(d,l-milchsäure-co-glykolsäure) 50:50 (Resomer RG502, Boehringer Ingelheim) eingebaut und zu sphärischen Mikropartikeln (RG502) verarbeitet. Die mit Antigen beladenen Mikropartikel wurden in einer 5% sterilen Lösung von Eilecithin (Ovothin 170, Lukas Meyer, D-Hamburg) durch Schütteln suspendiert. Diese Suspension wurde einer Gruppe von 8 BALB/c Mäusen zu je 0,5 ml subcutan injiziert. Die pro Maus injizierte Menge Antigen betrug 3x10 µg. Die Injektion wurde nach 16 Tagen (1. Booster) und nach 113 Tagen (2. Booster) wiederholt. Als Vergleich wurde eine zweite Gruppe von 8 BALB/c Mäusen mit der gleichen Menge Antigen in Inkomplettem Freund's Adjuvans (IFA) und nach dem gleichen Impfschema immunisiert. Die Antikörpertiter wurden mittels ELISA bestimmt.

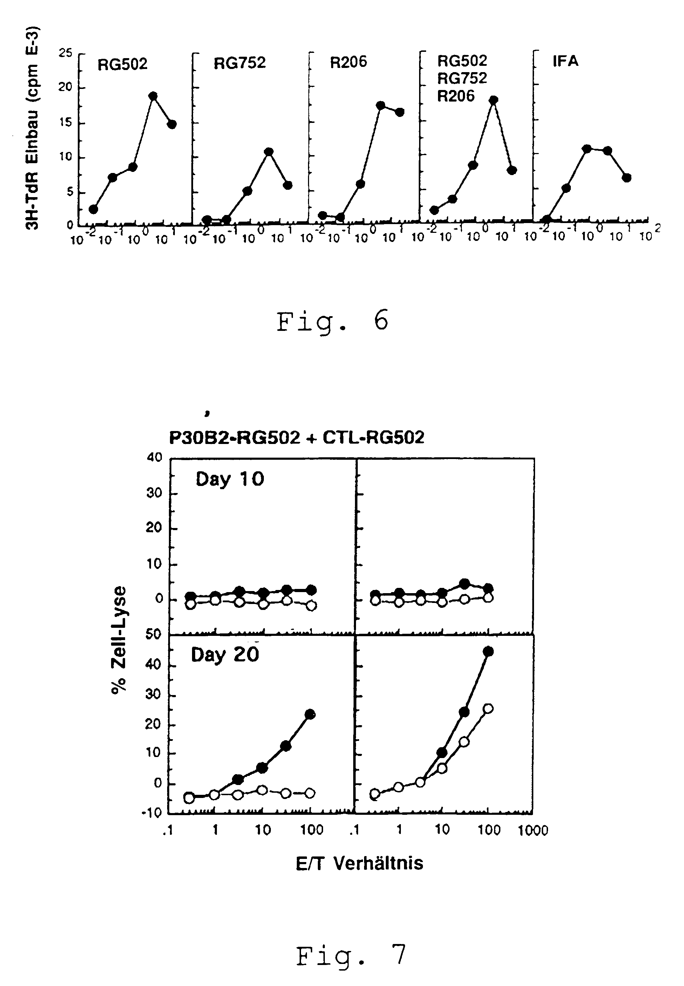

Fig. 5 zeigt den zum Beispiel 4 gehörenden zeitlichen Verlauf der Immunpotenzierung nach Booster Injektionen von RG502 im Vergleich zu IFA. Die mit RG502 und IFA erzielten Antikörpertiter steigen gleichermassen an. Das erfindungsgemässe Verfahren eignet sich demzufolge auch für die durch Boosten erzielte Immunpotenzierung.

Beispiel 5 beschreibt die Potenzierung der T_{H}-Lymphozyten-Proliferation auf das Multiple Antigen Peptid mit der Bezeichnung P30B2 gemäss Beispielen 1-4. P30B2 wurde analog Beispielen 1,2 und 3 in RG502, RG752, und R206 eingebaut und zu sphärischen Mikropartikeln mit unterschiedlich starker Quellbarkeit verarbeitet. Identische Mengen P30B2 enthaltende Mikropartikel wurden in einer 5% sterilen Lösung von Eilecithin (Ovothin 170, Lukas Meyer, D-Hamburg) durch Schütteln suspendiert. Diese Suspension wurde einer Gruppe von 8 BALB/c Mäusen zu je 0,5 ml subcutan injiziert. Die pro Maus injizierte Menge Antigen betrug 30 µg. Als Vergleich wurde eine zweite Gruppe von 8 BALB/c Mäusen mit der gleichen Menge Antigen in Inkomplettem Freund's Adjuvans (IFA) immunisiert. Die T-Zell-Proliferation in den Lymphknoten wurde in bekannter Weise bestimmt.

Fig. 6 zeigt die in Beispiel 5 beschriebene T-Lymphozyten-Proliferation 14 Tage nach Verabreichung verschiedener Mikrokapselformulierungen sowie einer IFA-Zubereitung. Es geht daraus hervor, dass alle Mikrokapselformulierungen, d.h. RG502 mit schneller Antigenfreigabe, RG752 mit mittelstark verlangsamter, und R206 mit stark verlangsamter Antigenfreigabe, sowie eine Mischung aller drei Mikrokapseltypen die T-Lymphozyten-Proliferation in einem mindestens vergleichbaren, z.T. stärkeren Masse potenziert als eine IFA-Zubereitung.

Beispiel 6 beschreibt das Auslösen einer cytotoxischen T-Lymphozyten-Reaktion auf ein T_{C}-Zell Epitop des Circumsporozoit Proteins von Plasmodium berghei (CTL 359A, Sequenz 252-260): 0,008 g CTL 359A wurden in 1,0 g Wasser gelöst und diese Lösung wurde anschliessend mit Hilfe eines Ultraschallgenerators in einer Lösung von 4,0 g Poly(d,l-milchsäure-co-glykolsäure) (Resomer 502, Boehringer Ingelheim) in 60,0 g Dichlormethan dispergiert. Mittels Sprühtrocknung wurde aus dieser Dispersion sphärische Mikropartikel hergestellt. Diese mit CTL 359A beladenen Mikropartikel wurden mit P30B2 beladenen Mikropartikeln, gemäss Beispiel 1, in einem CTL 359A : P30B2 Verhältnis von 1 : 10 gemischt, um die Immunantwort auf CTL zu erhöhen. Anschliessend wurde die Mischung der Mikrokapseln in einer 5% sterilen Lösung von Eilecithin (Ovothin 170, Lukas Meyer, D-Hamburg) durch Schütteln suspendiert. Diese Suspension wurde einer Gruppe von 2 BALB/c Mäusen zu je 0,5 ml subcutan injiziert. Die pro Maus injizierten Mengen betrugen: 4 µg CTL 359A und 40 µg P30B2. Die T_{C}-Zell Antwort wurde nach 10 und nach 20 Tagen mittels Zell-Lyse-Test bestimmt.

Fig. 7 zeigt die zum Beispiel 6 gehörende T_{C}-Lymphozyten-Antwort, die 10 und 20 Tage nach Immunisierung, bzw. nach der Verabreichung der Formulierungen, bestimmt wurde. Die prozentuale Zell-Lyse-Aktivität ist in Abhängigkeit des Effekt/Zielzellen-Verhältnisses E/T dargestellt. Die gleichzeitige Verabreichung von mikroverkapseltem T_{C}-Epitop und T_{H}-Epitop (P30B2 + 359A in RG502) induziert überraschenderweise eine signifikante T_{C}-Lymphozyten-Stimulation, die 20 Tage nach Verabreichung beobachtet werden kann. Besonders interessant erscheint der zeitliche Verlauf der T_{C}-Antwort, die im Gegensatz zur T_{H}- oder Antikörperantwort bedeutend längere Zeit benötigt.

Erfindungswesentlich ist, dass bioabbaubare sphärische Mikropartikel vorgeschlagen werden, welche die Immunantwort auf synthetische Antigene potenzieren. Durch Festlegen der physikalisch-chemischen Eigenschaften der verwendeten Biopolymere lässt sich diese Immunpotenzierung in ihrem Ausmass und zeitlichen Verlauf steuern. Das Verfahren ermöglicht es zudem, zusätzlich zur Antikörper- und T_{H}-Lymphozyten-Potenzierung auch die cytotoxischen T-Lymphozyten zu stimulieren. Das Ausmass der Immunpotenzierung ist mindestens vergleichbar mit der durch IFA-Zubereitungen erzielten Potenzierung und in ihrem zeitlichen Verlauf deutlich verlängert. Hiermit stehen pharmazeutische Zubereitungen zur Verfügung, die in der Immunisierung von Mensch und Tier gegen Krankheiten eingesetzt werden können, welche durch Viren, Bakterien, Protozoen oder Tumorzellen verursacht werden.

## Patentansprüche

1. Mikropartikel aus biokompatiblen und bioabbaubarem Material, in die ein Antigen eingelagert ist, dadurch gekennzeichnet, dass diese Mikropartikel mindestens ein cytotoxisches T-Zell Epitop enthalten und das biokompatible und bioabbaubare Material ein in wässerigen Medien und physiologischen Flüssigkeiten benetzbares quellbares unlösliches Biopolymeres ist.

2. Mikropartikel gemäss Anspruch 1, dadurch gekennzeichnet, dass sie neben mindestens einem cytotoxischen T-Zell Epitop ein Multiples Antigen Peptid (MAP) enthalten.

3. Mikropartikel gemäss einem der Ansprüche 1 oder 2, dadurch gekennzeichnet, dass das in wässerigen Medien und physiologischen Flüssigkeiten benetzbare quellbare und unlösliche Biopolymere aus Poly(milchsäuren), Poly(milch-co-glykolsäuren), Poly(hydroxybuttersäuren), Poly(hydroxybutter-co-valeriansäuren) und Poly(caprolactonen) ausgewählt ist.

4. Pharmazeutische Zubereitung zur parenteralen Applikation in Form einer Suspension von Mikropartikeln aus biokompatiblem und bioabbaubarem Material, in die ein Antigen eingebettet ist, in einem biokompatiblen und bioabbaubaren Dispersionsmedium, dadurch gekennzeichnet, dass
a) die Mikropartikel mindestens ein cytotoxisches T-Zell Epitop enthalten,
b) die Mikropartikel aus mindestens einem in wässerigen Medien und physiologischen Flüssigkeiten benetzbaren quellbaren unlöslichen Biopolymeren bestehen, und
c) das biokompatible und bioabbaubare Dispersionsmedium eine wässerige oder ölige Lösung von Lecithin oder wässerig-ölige Emulsionen mit Lecithin im Konzentrationsbereich von 0,1 bis 20%, oder eine aus einer Wasser-, Öl-, Tensid- und Kotensid-Komponente bestehende Mikroemulsion ist.

5. Pharmazeutische Zubereitung gemäss Anspruch 4, dadurch gekennzeichnet, dass die Mikropartikel neben mindestens einem cytotoxischen T-Zell Epitop ein Multiples Antigen Peptid (MAP) enthalten.

6. Pharmazeutische Zubereitung gemäss einem der Ansprüche 4 oder 5, dadurch gekennzeichnet, dass das in wässerigen Medien und physiologischen Flüssigkeiten benetzbare quellbare unlösliche Biopolymere aus Poly(milchsäuren), Poly(milch-coglykolsäuren), Poly(hydroxybuttersäuren), Poly(hydroxybutter-co-valeriansäuren) und Poly(caprolactonen) ausgewählt ist.

## Claims

1. Microparticles of a biocompatible and biodegradable material having an antigen embedded therein, characterised in that at least one cytotoxic T-cell epitope is included in said microparticles and that said biocompatible and biodegradable material is a biopolymer which is insoluble but wettable and swellable in aqueous media and physiological fluids.

2. Microparticles according to claim 1, characterised by having a multiple antigen peptide (MAP) in addition to at least one cytotoxic T-cell epitope included therein.

3. Microparticles according to either one of claims 1 and 2, wherein said biopolymer being insoluble but wettable and swellable in aqueous media and physiological fluids has been selected from poly(lactic acid), poly(lactic-co-glycolic acid), poly(hydroxybutyric acid), poly(hydroxybutyric-co-valeric acid), and poly(caprolactone).

4. A pharmaceutical composition for parenteral application, comprising microparticles of a biocompatible and biodegradable material having an antigen embedded therein, said microparticles being in suspension in a biocompatible and biodegradable dispersion medium, characterised in that
a) at least one cytotoxic T-cell epitope is included in said microparticles,
b) the microparticles are composed of at least one biopolymer which is insoluble but wettable and swellable in aqueous media and physiological fluids,
c) the biocompatible and biodegradable dispersion medium is an aqueous or oily solution of lecithin, or an aqueous-oily emulsion containing lecithin in a concentration range of 0,1 to 20 %, or a microemulsion comprising water, oil, surfactant and co-surfactant components.

5. The pharmaceutical composition according to claim 4, characterised in that said microparticles have a multiple antigen peptide (MAP) in addition to at least one cytotoxic T-cell epitope included therein.

6. The pharmaceutical composition according to either one of claims 4 and 5, characterised in that said biopolymer being insoluble but wettable and swellable in aqueous media and physiological fluids has been selected from poly(lactic acid), poly(lactic-co-glycolic acid), poly(hydroxybutyric acid), poly(hydroxybutyric-co-valeric acid), and poly(caprolactone).

## Revendications

1. Microparticules à base d'un matériau biocompatible et biodégradable, dans lesquelles est incorporé un antigène, caractérisées en ce que les microparticules contiennent au moins un épitope cytotoxique spécifique aux cellules-T et que le matériau biocompatible et biodégradable est un biopolymère insoluble, mais mouillable et gonflable dans les milieux aqueux et les fluides physiologiques.

2. Microparticules selon la revendication 1, caractérisées en ce qu'ils contiennent un peptide antigènique multiple (PAM) en plus d'au moins un épitope cytotoxique spécifique aux cellules-T.

3. Microparticules selon une des revendications 1 et 2, caractérisées en ce que le biopolymère insoluble, mais mouillable et gonflable dans les milieux aqueux et les fluides physiologiques est choisi parmi le poly(acide lactique), le poly(acide lactique-co-glycolique), le poly(acide hydroxybutyrique), le poly(acide hydroxybutyrique-co-valérique) et le poly(caprolactone).

4. Composition pharmaceutique pour application parentérale, comprenant des microparticules à base d'un matériau biocompatible et biodégradable, dans lesquelles est incorporé un antigène, ces microparticules étant en suspension dans un milieu de dispersion de nature biocompatible et biodégradable, caractérisée en ce que:
a) les microparticules contiennent au moins un épitope cytotoxique spécifique aux cellules-T;
b) les microparticules sont composées d'au moins un biopolymère insoluble, mais mouillable et gonflable dans les milieux aqueux et les fluides physiologiques; et
c) le milieu de dispersion de nature biocompatible et biodégradable est une solution aqueuse ou huileuse de lécithine, ou une émulsion aqueuse-huileuse contenant de 0,1 à 20% en lécithine, ou une microémulsion constituée d'eau, d'huile, de surfactif et de co-surfactif.

5. Composition pharmaceutique selon la revendication 4, caractérisée en ce que les microparticules contiennent un peptide antigènique multiple (PAM) en plus d'au moins un épitope cytotoxique spécifique aux cellules-T.

6. Composition pharmaceutique selon une des revendications 4 et 5, caractérisée en ce que le biopolymère insoluble mais mouillable et gonflable dans les milieux aqueux et les fluides physiologiques, est choisi parmi le poly(acide lactique), le poly(acide lactique-coglycolique), le poly(acide hydroxybutyrique), le poly(acide hydroxybutyrique-co-valérique), et le poly(caprolactone).
